(19)

Europäisches
Patentamt

European
Patent Office

Office européen
des brevets

(11) **EP 2 889 028 A1**

(12) **EUROPEAN PATENT APPLICATION**

(43) Date of publication:
01.07.2015  Bulletin 2015/27

(21) Application number: 15151038.5

(22) Date of filing: 23.06.2009

(51) Int Cl.:
*A61K 9/00* *(2006.01)*   *A61K 47/24* *(2006.01)*
*A61K 47/10* *(2006.01)*   *A61K 31/00* *(2006.01)*
*A61K 9/08* *(2006.01)*

(84) Designated Contracting States:
AT BE BG CH CY CZ DE DK EE ES FI FR GB GR
HR HU IE IS IT LI LT LU LV MC MK MT NL NO PL
PT RO SE SI SK TR

(30) Priority: 24.06.2008  US 75067 P
12.08.2008  EP 08162240

(62) Document number(s) of the earlier application(s) in
accordance with Art. 76 EPC:
09769218.0 / 2 293 777

(71) Applicant: Intervet International B.V.
5831 AN  Boxmeer (NL)

(72) Inventors:
• Freehauf, Keith
Summit, NJ New Jersey NJ 07901 (US)

• Meadows, Cheyney
Summit, NJ New Jersey 07901 (US)
• Sheehan, John Gerard
Summit, NJ New Jersey 07901 (US)

(74) Representative: Stumm, Karin
Merck Sharp & Dohme Ltd.
Hertford Road
Hoddesdon, Hertfordshire
EN11 9BU (GB)

Remarks:
This application was filed on 14-01-2015 as a
divisional application to the application mentioned
under INID code 62.

(54) **Pharmaceutical transdermal compositions and method for treating inflammation in cattle**

(57)    Novel transdermal preparations combining a non-steroidal antiinflammatory drug (NSAID) selected from groups such as oxicams (for example, meloxicam), propionic acids (for example, ketoprofen) and anthranilic acids (for example, tolfenamic acid), are disclosed. Methods for using and administering such preparation in the treatment of inflammatory conditions in bovines are also disclosed.

EP 2 889 028 A1

**Description**

**FIELD OF THE INVENTION**

The present invention relates to compositions and methods for the treatment of inflammation in animals. More particularly, the invention relates to transdermal administration of non-steroidal anti-inflammatory compounds (NSAID) to non-human animals.

**BACKGROUND OF THE INVENTION**

All patents, applications, publications, test methods, and other materials cited herein are incorporated by reference.

Inflammation is a process that occurs in response to injury or other abnormal stimulation by physical, chemical, or biological agents, with the purpose of helping to overcome the abnormal stimulus. Inflammation involves local tissue reactions and morphologic changes, destruction or removal of injurious material, and the initiation of repair and/or healing. Cardinal signs of active inflammation include redness, heat, swelling, pain, and reduction or loss of function; these signs can present locally and/or systemically.

While the purpose of an inflammatory response is to help the host overcome an abnormal stimulus, inflammatory episodes can have deleterious effects. In the short-term, febrile or painful animals may have reduced feed and water intake, which can create the risk of developing problems related to a negative energy balance or dehydration. Furthermore, some inflammatory episodes can leave long-lasting residual damage, scarring, and reduced functionality.

For example, bovine respiratory disease (BRD) occurs in both dairy and beef cattle and is one of the leading causes of economic loss to the cattle industry throughout the world. Economic losses are attributable to excessive mortality, treatment and prevention costs, and decreased productivity - dairy cattle with clinical or sub-clinical BRD do not gain weight or produce milk as well as healthy animals, and beef cattle with BRD gain less weight, have reduced feed efficiency and often produce a lower grade carcass at slaughter. A direct correlation between pulmonary lesions observed at slaughter and reduced weight gains has been established in cattle with sub-clinical BRD infections. The etiologic agents of BRD include bacterial organisms such as *Mannheimia haemolytica, Pasteurella multocida* and *Histophilus somni.* However, in BRD infections, the pulmonary damage that results in death or morbidity is often due to an excessive host inflammatory response to the invading pathogens. In the short term, febrile, painful animals eat and drink less. Furthermore, long-term damage to host tissues occurs, resulting in long-term declines in productivity even after BRD infection has resolved.

Bovine mastitis is considered to be the most costly production disease faced by the dairy industry, costing hundreds of millions of dollars per year. Bovine mastitis is typically caused by infectious agents such as *Staphylococcus aureus, Streptococcus* species, and *Escherichia coli.* In response to infection, the mammary gland undergoes an inflammatory process, characterized by warmth, pain, redness, swelling, and impaired function. The affected animal often develops a fever and eats and drinks less. There is a transient decrease in milk production during the acute inflammatory stage, and subsequent milk yield for the remainder of the lactation is reduced as a result of residual inflammatory damage.

In addition to cattle, other species are similarly susceptible to short-term and long-term effects of inflammatory episodes induced by a variety of causes. Regardless of species or causative agent, the damage brought about by inflammation evolves as neutrophils and other inflammatory cells destroy affected tissues. As cell membranes are damaged, arachidonic acid is released. Arachidonic acid is the substrate for the formation of various prostaglandins and other eicosanoids. The release of these biologically active substances is critical to driving the inflammatory response that results in additional pain, inflammatory damage and lesions. Non-steroidal anti-inflammatory drugs (NSAIDs) effectively modulate inflammation by disrupting the arachidonic acid cascade.

Use of NSAIDs is a cornerstone of management of pain and inflammatory processes in human and veterinary medicine. Regardless of the species or organ system affected or the cause, pharmacologic modulation of inflammation offers important quality of life benefits to painful or febrile animals, allowing the affected animal to eat and drink and thus increase the potential for recovery. Furthermore, use of NSAIDs helps to reduce excessive damage that results in long-term reduction of functionality, thus bringing economic benefits to livestock producers.

Based on structure, most commercially available veterinary NSAIDs can be divided into 2 broad classes - carboxylic acid and enolic acid derivatives. These classes can be further divided into groups based on similar molecular structures. NSAIDs within a group - those that share similar molecular will tend to have similar characteristics and tolerability. The main groups of enolic acids are the pyrazolones (phenylbutazone, oxyphenbutazone, and ramifenazone) and the oxicams (meloxicam, piroxicam, and tenoxicam). Carboxylic acid groups include the salicylates (aspirin), propionic acids (ibuprofen, naproxen, carprofen, ketoprofen, and vedaprofen), anthranilic acids (tolfenamic and meclofenamic acids), phenylacetic acids (acetaminophen), aminonicotinic acids (flunixin), and indolines (indomethacin).

Currently there are several NSAIDs commercially available for cattle and licensed in several countries i.e.

flunixin meglumine (Finadyne® Injection, Schering-Plough Animal Health), ketoprofen (Ketofen® 10%, Merial), meloxicam (Metacam®, Boehringer Ingelheim) and tolfenamic acid (Tolfine®, Vetoquinol). All products are licensed for being administered parenterally.

Conventionally, NSAID products are administered to animals parenterally by injection. Flunixin meglumine is e.g. currently formulated for intravenous or intramuscular injection, meloxicam for intravenous or subcutaneous injection, and ketoprofen and tolfenamic acid for intravenous or intramuscular injection. This means the administration is performed by injection, using a syringe and needle.

In order to properly inject a drug to a bovine animal, like cattle, it is necessary to restrain the animal properly in order to avoid any injury to the animal and the farmer or veterinarian. Furthermore injection through the skin may lead to injection site reaction, i.e. local inflammation reactions in the tissue circumventing the injection site. Such tissue reaction might persist until the slaughter of the animal and under certain circumstances will be still present in the meat for human consumption. In addition, any injection to big herds of cattle animals under practice conditions bears the risk, that infections are transferred from one animal to the other by the needle used. Consequently, there is a need for alternative administration methods of NSAID drugs that avoid these drawbacks and are safe to use, and avoid such risk, injury and injection site reactions.

Thus, there is a need for an improved formulation and method of administration, such as a formulation for transdermal drug delivery, which addresses these problems. One difficulty faced, however, when attempting to arrive at a transdermal formulation is the fact that the skin has been described as a "black box" with regard to drug delivery. This is due to the lack of knowledge in the mechanisms of drug penetration through the epidermis and partitioning into the underlying layers. Thus far, the boundaries for such properties have not been defined; making it very difficult to predict what compounds can be delivered transdermally.

Transdermal systems effective for delivering one compound are almost always ineffective with other compounds. Also, systems and devices that work in one species are usually ineffective in other species. Furthermore, due to the presence of the stratum corneum barrier, the mass transfer through the skin is usually too slow for rapid, massive systemic absorption. This explains why very few, if not any, of the commercially available transdermal products for human use are designed for immediate drug delivery. Transdermal drugs that work in humans are not always suitable for fur bearing animals, especially for bovine animals.

Accordingly, there is a need for stable, transdermal liquid preparation that offers a way for handlers to safely and conveniently administer NSAID compounds to animals in need thereof to ameliorate pain and inflammation, while minimizing the pain and stress to the animal associated with treatment and the potential for injection site tissue damage.

## SUMMARY OF THE INVENTION

The present invention fulfills this need by providing improved preparations and methods for the delivery of NSAID compounds selected from the group consisting of oxicams, propionic acids, and anthranilic acids, or pharmaceutically acceptable salts thereof to animals, especially bovine animals.

In one embodiment the NSAID compound of the oxicams group is meloxicam, of the propionic acids group is ketoprofen and of the anthranilic acids group is tolfenamic acid.

Accordingly, there are disclosed pharmaceutically acceptable preparations for transdermal administration to animals and methods for the use thereof. Such preparations comprise one or more of a NSAID compound selected from the oxicams group, the propionic acid group and the anthranilic acid group or pharmaceutically acceptable salts thereof, a pharmaceutically acceptable carrier system comprising at least one solvent, and at least one dermal penetration enhancer. In optional aspects of the invention, the transdermal liquid preparations can include a stabilizing or viscosity lowering agent, such as water, ethanol, isopropanol, propylene glycol, dimethylisosorbide, triacetin, or glycerol.

One preferred aspect of the invention includes a transdermal liquid preparation containing:

a) a NSAID compound selected from one of the following groups:

oxicams, propionic acids and anthranilic acids or
pharmaceutically acceptable salts thereof;

b) at least one dermal penetration enhancer; and

c) at least one solvent.

Within the first and second aspect of the invention, the dermal penetration enhancer can be present in an amount from about 2% to about 90% of the transdermal liquid preparation. Preferred dermal penetration enhancers include menthol, xylene, D-limonene, isopropyl myristate, propylene glycol dicaprylate/dicaprate, decanoic acid, decyl alcohol, oleic acid, or mixtures thereof.

The amount of the non steroidal anti-inflammatory (NSAID) compound selected from oxicams, propionic acids and anthranilic acids included in the transdermal liquid preparations described herein can be present in an amount from about 0.5 to about 40% by wt., while the amount of the solvent can broadly be from about 10 to about 90% by wt. Preferred solvents useful in the present invention include pyrrolidone solvents such as 2-pyrrolidone or N-methyl-2-pyrrolidone, ethyl lactate, and glycol ethers such as ethylene glycol monoethyl ether, diethylene glycol monoethyl ether, dipropylene glycol monoethyl ether, ethanol, isopropyl alcohol, and benzyl alcohol.

In another aspect of the invention, there are provided methods of treating pain and inflammatory conditions. Some of these methods include administering an effective amount of a transdermal preparation as described above to an animal, like a mammal such as a bovine animal (e.g. cow) in need thereof.

The present preparation can also optionally include other NSAIDs besides oxicams, propionic acids and anthranilic acids, as well as other active pharmaceutical ingredients such as anti-microbials, hormones for reproduction, growth enhancement, or other physiologic intervention, anxiolytic compounds, antihistamines, immune stimulants, vaccines and the like, for example.

In another aspect of the invention, there are provided methods of administering the transdermal NSAID liquid preparation incorporating the transdermal liquid preparation into a press-in bottle application device, and administering an effective amount of the transdermal liquid preparation to an animal in need thereof.

With the foregoing and other objects, advantages and features of the invention that will become apparent hereinafter, the nature of the invention may be more clearly understood by reference to the following detailed description of the invention and the appended claims.

**BRIEF DESCRIPTION OF THE FIGURES**

Figure 1 is a graph showing the results of one of the tests carried out in the experiment described in Example 2, wherein the mean plasma concentration of meloxicam was measured following a single transdermal or injectable dose in cattle.

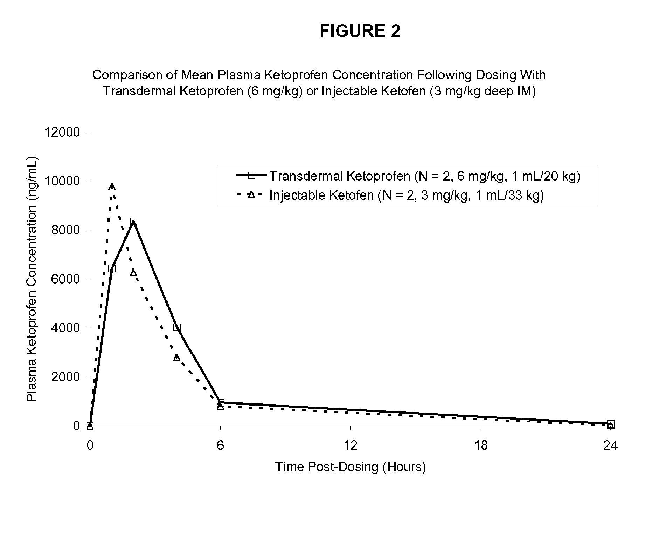

Figure 2 is a graph showing the results of one of the tests carried out in the experiment described in Example 2, wherein the mean plasma concentration of ketoprofen was measured following a single transdermal or injectable dose in cattle.

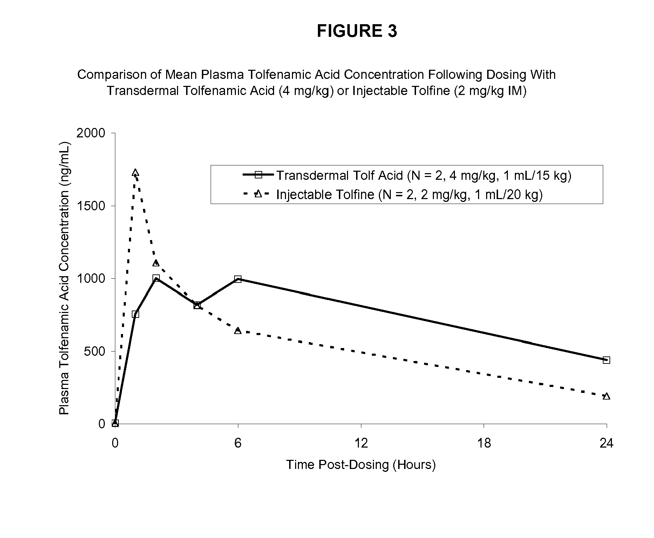

Figure 3 is a graph showing the results of one of the tests carried out in the experiment described in Example 2, wherein the mean plasma concentration of tolfenamic acid was measured following a single transdermal or injectable dose in cattle.

**DETAILED DESCRIPTION OF THE INVENTION**

It has been found that effective concentrations of a NSAID compound selected from oxicams, propionic acids, or anthranilic acids or pharmaceutically acceptable salts thereof in the systemic circulation for the purpose of providing systemic anti-inflammatory activity can be achieved by the transdermal route of administration. This can encompass various types of delivery including pour-on, spot-on, spray, dip, wipe, etc.

The present invention relates to an NSAID product for providing systemic anti-inflammatory (including anti-pyrexia and analgesia) activity for animals, especially bovine mammals such as cows. The present invention demonstrates that, through improved compositions and methods of delivery a NSAID compound selected from the following groups: oxicams (for example, meloxicam), propionic acids (for example, ketoprofen), or anthranilic acids (for example, tolfenamic acid) can effectively diffuse through the skin and further partition into the underlying layers for rapid absorption. It was discovered that the pharmacokinetic parameters of the present invention are comparable to those obtained by the counterpart injectable formulations. The pharmacokinetic data shows high bioavailability and efficiency of skin barrier penetration, as well as tissue partitioning from the current formulations.

As used herein, the following terms, unless otherwise indicated, shall be understood to have the following meanings:

Non-steroidal anti-inflammatory drugs, usually abbreviated to NSAIDs, are drugs with analgesic, antipyretic and, in higher doses, anti-inflammatory effects - they reduce pain, fever and inflammation. The term "non-steroidal" is used to distinguish these drugs from steroids, which (among a broad range of other effects) have a similar eicosanoid-depressing, anti-inflammatory action. NSAIDs can be classified based on their chemical structure. NSAIDs within a group - those that share similar molecular structures - will tend to have similar characteristics and tolerability.

Propionic acids (2-Arylpropionic acids (profens)) are NSAIDs having a free-CH (CH3) COOH or- CH2CH2COOH or a pharmaceutically acceptable salt group, such as-CH (CH3) COO- Na+ or CH2CH2COO-Na+, which are typically attached directly or via a carbonyl functionality to a ring system, preferably an aromatic ring system. Examples of propionic acid derivatives include ibuprofen, naproxen, benoxaprofen, naproxen sodium, flurbiprofen, fenoprofen, fenbuprofen, ketoprofen, indoprofen, pirprofen, carprofen, oxaprofen, pranoprofen, microprofen, tioxaprofen, suprofen, alminoprofen, tiaprofenic acid, fluprofen and bucloxic acid. Ketoprofen is 2-(3-benzoylphenyl)propanoic acid (CAS 22071-15-4).

Anthranilic acids (N-Arylanthranilic acids) are based on the molecule fenamic acid, which is formed by two carbon rings attached in the middle with one nitrogen atom, and with one carboxyl group (COOH) on one of the rings. Anthranilic acids are e.g. mefenamic acid, tolfenamic acid, flufenamic acid, and meclofenamic acid. Tolfenamic acid is 2-[(3-chloro-2-methylphenyl)amino]benzoic acid). (CAS13710-19-5).

Oxicam NSAIDs belong to the class of drugs called enolic acid derivatives. Enolic acid is a carbon- and hydroxyl-containing molecule (C=C-OH) made from carboxylic acid. Structurally related oxicams are Meloxicam, Piroxicam, Tenoxicam, Droxicam, Lornoxicam Meloxicam is 4-hydroxy-2-methyl-N-(5-methyl-2-thiazolyl)-2H-1,2-benzothiazine-3-carboxamide-1,1-dioxide (CAS 71125-38-7).

"transdermal application" and/or "transdermal liquid preparation" is intended to encompass all such methods known for allowing a pharmaceutically active ingredient to be delivered at least partially through the skin, usually by applying the composition containing the active ingredient and formulation excipients externally to the surface, i.e. skin, fur, etc. of an animal and allowing sufficient time for absorption through the dermal layers of the animal being treated. Methods of administration include pour-on, spot-on, spray, dip, wipe, or other methods apparent to those skilled in the art;

"pour-on" is intended to encompass routes of administration in which an effective amount of a suitable pharmaceutically active ingredient is externally applied to a localized region, allowing for diffusion of an effective amount of the pharmaceutically active ingredient to the affected area(s) or systemic distribution or a region which will facilitate delivery of the pharmaceutically active ingredient to the affected area(s) or systemic distribution;

"composition" "formulation" and/or "preparation" is intended to encompass a product comprising the specified ingredients disclosed herein in the specified amounts disclosed herein, as well as any product which results, directly or indirectly, from combination of the specified ingredients disclosed herein in the specified amounts disclosed herein; and

"effective amount" is a dose required to alleviate a particular symptom of an infection or disease.

In accordance with a first aspect of the invention, the transdermal liquid preparation contains a therapeutically effective amount of a NSAID compound selected from the following groups: oxicams (for example, meloxicam), propionic acids (for example, ketoprofen), or anthranilic acids (for example, tolfenamic acid) or a pharmaceutically acceptable salt thereof, a dermal penetration enhancer, and a solvent.

In the preparations of the invention, the concentration of the NSAID compound can be from about 0.5% to about 40% by weight of the transdermal liquid preparation, or particularly from about 1% to about 20% by weight, or particularly with amounts being from about 2% to about 15. The NSAID compound selected from the oxicams (for example, meloxicam), propionic acids (for example, ketoprofen), or anthranilic acids (for example, tolfenamic acid) can be introduced into the preparation as a pharmaceutically acceptable salt, in which case the concentration of the salt would be adjusted in order to maintain the preferred NSAID compound concentration.

The transdermal liquid preparation of the invention also includes a dermal penetration enhancer. In particular embodiments of the invention, the dermal penetration enhancer is present in amounts from about 2 to about 90% w/v of the transdermal liquid preparation, particularly from about 5 to about 80% w/v or particularly from about 10 to about 70% w/v.

Non-limiting examples of a suitable dermal penetration enhancer include, but are not limited to, terpenoids such as menthol, camphor, d-limonene, nerolidol, 1-8 Cineole and mixtures thereof, saturated or unsaturated fatty acid esters or diesters of propylene glycol or esters ,diesters or triesters of glycerol, saturated or unsaturated fatty acids, saturated or unsaturated fatty alcohols, xylene, isopropyl myristate, or mixtures thereof. Particularly, the dermal penetration enhancer is menthol, propylene glycol dicaprylate/dicaprate and/or xylene and/or D-limonene and/or isopropyl myristate.

In one embodiment the transdermal liquid preparation of the invention includes two or more dermal penetration enhancer.

In particular embodiments of the invention, the first dermal penetration enhancer is present in amounts from about 2 to about 30% w/v of the transdermal liquid preparation, particularly from about 3 to about 25% w/v or particularly from about 5 to about 20% w/v.

Non-limiting examples of a suitable first dermal penetration enhancer include, but are not limited to, terpenoids such as menthol, camphor, d-limonene, nerolidol, 1-8 Cineole, propylene glycol dicaprylate/dicaprate, decanoic acid, decyl alcohol, oleic acid, and mixtures thereof. Particularly, the first dermal penetration enhancer is menthol.

The optional second dermal penetration enhancer is particularly present in an amount from about 2 to about 90% w/v of the transdermal liquid preparation, particularly from about 5 to about 80% w/v, or more particularly from about 10 to about 70% w/v.

Non-limiting examples of a suitable optional second dermal penetration enhancer include, but are not limited to, a second terpenoid, saturated or unsaturated fatty acid esters or diesters of propylene glycol or esters ,diesters or triesters of glycerol, saturated or unsaturated fatty acids, saturated or unsaturated fatty alcohols and mixtures thereof.

Particularly, the optional second dermal penetration enhancer is xylene, D-limonene, isopropyl myristate, propylene glycol dicaprylate/dicaprate, decanoic acid, decyl alcohol, oleic acid or mixtures thereof. Particularly, the optional second dermal penetration enhancer is propylene glycol dicaprylate/dicaprate and/or xylene and/or D-limonene and/or isopropyl myristate and/or mixtures thereof.

In one particular preparation of the invention, the first dermal penetration enhancer is menthol, and the optional second dermal penetration enhancer is propylene glycol dicaprylate/dicaprate and/or xylene and/or D-limonene and/or isopropyl myristate and/or mixtures thereof.

In one embodiment the penetration enhancer is a combination of propylene glycol dicaprylate/dicaprate and menthol.

In case more than one penetration enhancer is employed the ratio of the first dermal penetration enhancer to the optional second dermal penetration enhancer is from about 4:1 to about 1:20.

The transdermal liquid preparation of the invention also includes a solvent. In particular formulations of the invention, the solvent is present in an amount from about 5 to about 90% by weight of the transdermal liquid preparation, particularly, from about 10 to about 80% by weight.

Non-limiting examples of a suitable solvent include, but are not limited to, solvents e.g. a pyrrolidone solvent, such as 2-pyrrolidone, N-methyl-2-pyrrolidone, and/or mixtures thereof, and glycol ethers such as ethylene glycol monoethyl ether, diethylene glycol monoethyl ether, or dipropylene glycol monoethyl ether, N,N-dimethylacetamide, N,N-dimethylformamide, dimethyl sulfoxide (DMSO), acetone, glycerol formal, ethyl lactate, or mixtures thereof. Particularly, the solvent is a pyrrolidone solvent such as 2-pyrrolidone, N-methyl pyrrolidone (NMP), and a glycol ether such as diethylene glycol monoethyl ether (DEGMEE) or mixtures thereof and the like.

Non-limiting examples of other solvents include, but are not limited to, water, ethanol, isopropanol, 1,2-propanediol, glycerin, benzyl alcohol, dimethylisosorbide, triacetin, propylene glycol, ethyl lactate, glycol ethers such as ethylene glycol monoethyl ether, diethylene glycol monoethyl ether (DEGMEE), or dipropylene glycol monoethyl ether, and polyethylene glycols (PEG) having an average molecular weight between about 200 and 1000. In particular, solvents include isopropyl alcohol, benzyl alcohol, and PEG having an average molecular weight between about 200 and about 1000, triacetin, dimethylisosorbide, ethanol, and water, and combinations thereof.

The addition of one or more of additional other solvents may be desirable to alter the viscosity of the formulation in order to provide a product with appropriate characteristics for transdermal application.

The transdermal liquid preparation of the invention can also optionally include a second pharmaceutically active compound, or other therapeutic classes of drugs such as anti-microbials, anti-inflammatory agents, oxytocin, hormones for reproduction, growth enhancement compounds, physiologic intervention compounds, anxiolytic compounds, antihistamines, immune stimulants, and vaccines and the like, for example. As will be appreciated by those of ordinary skill, a wide variety of pharmaceutically active compounds/agents can be included with the NSAID compound based transdermal formulations described herein. The only limitation on the type of pharmaceutical agent which can be included is that the second agent must not significantly interact with or significantly diminish the activity of the NSAID compound or pharmaceutically acceptable salt being transdermally administered.

A non-limiting list of suitable pharmaceutically active compounds include those falling in the categories of anti-inflammatory agents, such as NSAIDs and corticosteroids, antibiotics, anti-pyretics, analgesics, etc. and the like. In one particular aspect, the transdermal formulations will include an antibiotic such as a fluorine-containing analog chloramphenicol and thiamphenicol, such as florfenicol. Examples of such compounds, and methods for their manufacture, are described and claimed in U.S. Patent No. 4,235,892.

Suitable antimicrobials include, but are not limited to, compounds from classes such as aminoglycosides, beta-lactams, cephalosporins, floroquinolones, lincosamides, macrolides, sulfonamides and potentiated sulfonamides, tetracyclines, and fluorine-containing analogs of chloramphenicol. Suitable growth enhancing agents include, without limitation, somatotropin and zeranol. Suitable anxiolytic compounds include, without limitation, NOP-1 receptor agonists, NK-1 receptor antagonists, benzodiazepines, and phenothiazines. Suitable antihistamines include, without limitation, diphenhydramine and tripelennamine.

Other ingredients can be added to the present composition, as desired. Such ingredients include preservatives, chelating agents, antioxidants, and viscosity modifying agents. Exemplary preservatives include without limitation methyl p-hydroxybenzoate (methylparaben) and propyl p-hydroxybenzoate (propylparaben), added in an appropriate quantity known to one skilled in the art. Exemplary chelating agents include without limitation edetate disodium and EDTA. Exemplary antioxidants include without limitation butylated hydroxyanisole, ascorbic acid, and sodium monothioglycerol, added in an appropriate quantity known to one skilled in the art. Suitable viscosity modifying agents include, without limitation, water, ethanol, isopropanol, propylene glycol, dimethylisosorbide, triacetin, or glycerol, added in an appropriate quantity known to one skilled in the art.

In order to prevent degradation of any of the active ingredients in the formulations of the present invention, the addition of at least one stabilizer has been found to be advantageous. In order to prevent degradation of any of the active ingredients in the formulations of the present invention, a pH adjusting agent has been found to be advantageous.

The amount of the active agent(s) or any other excipients may be varied to alter the dose volume delivered or the physical properties of the formulation. The amount of the second pharmaceutically or therapeutically active agent will depend on transdermal bioavailability and pharmacologic synergy with other actives in the formulation and will be titrated to effect.

In some particular embodiments, the transdermal preparations in accordance with the invention have a similar plasma profile to that observed with the commercially available injectable products for cattle containing meloxicam, ketoprofen or tolfenamic acid as active ingredient.

It will also be appreciated that the present invention encompasses, in one aspect, methods of treating inflammation by administering, the preparation as described above to an animal, especially a bovine animal by transdermal administration.

The transdermal liquid preparation according to the invention has an unexpected high absolute systemic bioavailability. Bioavailability is a measurement of the extent of a therapeutically active drug that reaches the systemic circulation and is available at the site of action. It is expressed as the letter $F$. Absolute bioavailability measures the availability of the active drug in systemic circulation after non-intravenous administration (i.e., after oral, transdermal, subcutaneous administration).

The absolute bioavailability is the dose-corrected area under curve (AUC) non-intravenous divided by AUC intravenous. For example, the formula for calculating $F$ for a drug administered by the oral route (po) is given below.

$$F = \frac{[AUC]_{po} * dose_{IV}}{[AUC]_{IV} * dose_{po}}$$

In one embodiment the preparation according to the invention has an absolute systemic bioavailability of at least 10% after transdermal administration. In particular the preparation according to the invention has an absolute systemic bioavailability of 10 - 20%. In a preferred embodiment the absolute systemic bioavailability is higher than 20%.

The preparation can be applied in a variety of ways, such as a pouring, spraying, or wiping on to any area of the animal's skin, including the back, ears, or udder, preferably the back of the animal.

The present invention also includes a transdermal preparation for the treatment of inflammatory conditions in an animal. Particularly, the transdermal preparation comprises from about 2% to about 90% by wt of a dermal penetration enhancer, from about 0.05% to about 40% of the NSAID compound or a pharmaceutically acceptable salt thereof, from about 10% to about 90% of a solvent. In addition to greater convenience and ease of use, it is believed that a single daily administration of a transdermal product in accordance with the present invention will promote humane animal care by reducing the number of injections needed to treat animals and providing rapid relief of disease symptoms. By reducing the number of injections, manpower costs also may be significantly reduced.

In a particular method of preparing the composition of the present invention, the vehicle(s) or a portion of the vehicle(s), are added to the compounding vessel, followed by the remaining excipients and the actives. The mixture is mixed until all solids are dissolved. An additional solvent to bring the composition to final volume may be added if needed. Additives, such as those listed above, may also be included in the vessel and mixed into the formulation. The order of addition of the above vehicles, excipients, solvents and additives is not critical.

The preparations may be administered once daily or divided into multiple doses. In some circumstances, daily doses will be required to treat the animal. The precise dose will depend on the stage and severity of the condition being treated, and the individual characteristics of the animal species being treated, as will be appreciated by one of ordinary skill in the art.

The preparations of the present invention may be administered in a press in bottle insert application device (PIBA) to an animal in need thereof. Such a device allows a health care professional to easily dispense liquids from stock bottles into (oral) syringes. In administering the composition, the professional opens the bottle and presses the plastic adapter into the opening of the bottle and then attaches the oral syringe to the port of the adapter. Next, the professional may withdraw the dose of medication from the bottle and administer the dose. Then the cap can be replaced on the bottle to be used later. Presently, animal pour-on products generally require administering larger volumes of a composition, thus, the above-described method of administration is not appropriate. Therefore, present pour-on products are either administered in a dosing gun or a dosing cup. Such methods of administration prove difficult to accurately deliver small volumes of medication. Thus, the method of administration of the present invention using the PIBA application system allows for more accurate and convenient administration of the presently claimed pour-on liquid preparation.

The preparations according to the present invention are particularly useful for bovine animals. In this specification, bovine animals are ruminant mammals of the genus *Bos* and include, but are not limited to, cattle, steers, heifers, cows (lactating and non-lactating), calves, bulls, and also buffalo

In addition to the treatment of BRD, the compositions of this invention are also suitable for the treatment of other conditions associated with inflammation such as footrot, acute mastitis, pinkeye (infectious keratoconjunctivitis), acute pneumonia, metritis and enteritis in bovine animals. The dosage regimen for treatment of such diseases should be appropriate for the species and condition being treated.

Mastitis is a complex disease that occurs in lactating females, and is of particular economic importance in dairy cows and goats. Several pathogenic agents may be involved, including *Staphylococcus aureus, Escherichia coli,* and *Streptococcus* species. The acute form of mastitis has a sudden onset, the udder is enlarged, hot to the touch and tender; and usually the affected animal will have a fever. If not treated promptly, the udder may be permanently damaged and milk production may be decreased or lost.

Pinkeye is an acute infectious disease of cattle, sheep and other animals that is characterized by inflammation of the tissues of the eye, accompanied by nasal discharge, lacrimation and copious ocular discharge. Affected animals may display extreme discomfort, resulting in decreased feed intake and subsequent reduction in body weight gain and/or a drop in milk production. In extreme cases, permanent blindness occurs. The disease, which is caused by *Moraxella bovis* in cattle, is widespread, especially among range and feedlot cattle, the cure of which is of great economic importance to the cattle industry.

Footrot (interdigital phlegmon) is an acute infection of the interdigital space that occurs throughout the world in both beef and dairy cattle. *Fusobacterium necrophorum* is the major cause of footrot, although other organisms, including *Bacteroides melaninogenicus,* can be involved. The major symptoms include pain, severe lameness, fever, anorexia, and reduced milk production. Currently, footrot is treated by antibiotic therapy. Recommended therapy can involve treatment for up to five days. The use of the preparations of the present invention would be a useful adjunct therapy because the NSAID would reduce the inflammation caused by footrot and make the animal feel better.

## **EXAMPLES**

The materials and methods of the present invention are further illustrated by the examples which follow. These examples are offered to illustrate, but not to limit, the claimed invention.

EXAMPLE 1

Composition of transdermal preparations of meloxicam, ketoprofen, and tolfenamic acid tested in cattle.

| | Concentration % w/v | | |
|---|---|---|---|
| Ingredient | Transde rmal Meloxicam Batch 86361-19 | Transdermal Ketoprofen Batch 86361-18 | Transdermal Tolfenamic Acid Batch 86361-42 |
| Meloxicam | 2.0 | --- | --- |
| Ketoprofen | --- | 12.0 | --- |
| Tolfenamic Acid | --- | --- | 6.0 |
| L-menthol | 10.0 | 10.0 | 10.0 |
| Miglyol 840 | 20.0 | 20.0 | 20.0 |
| 2-pyrrolidone | 50.0 | --- | |

(continued)

| Ingredient | Concentration % w/v | | |
| --- | --- | --- | --- |
| | Transde rmal Meloxicam Batch 86361-19 | Transdermal Ketoprofen Batch 86361-18 | Transdermal Tolfenamic Acid Batch 86361-42 |
| Isopropyl alcohol | 13.0 | --- | 44.0--- |
| NMP | --- | --- | 20.0 |
| DEGMEE | --- | 54.0 | --- |

In order to prepare the compositions of the Example, the vehicle(s) or a portion of the vehicle(s), are added to the compounding vessel, followed by the remaining excipients and the actives. The combination is mixed until all solids are dissolved. Although not included herein, additives, such as those mentioned in the detailed description, are also included in the vessel and mixed into the formulation. The order of addition was not critical.

EXAMPLE 2

Experiments to Measure Pharmacokinetics of Meloxicam, Ketoprofen, and Tolfenamic Acid of Formulations Described in Example 1 in Cattle

The post-dosing plasma concentration of the active components of the formulations described in Example 1 was assessed in a research study

3 pairs of cattle were randomly assigned to transdermal treatment with one of the formulations described in Example 1 on Day 0 of the experiment. One week after transdermal treatment (Day 7 of the experiment), the cattle were treated with an injectable formulation of the same active used on Day 0. Injectable products were given in accordance with label instructions for cattle. Blood samples for concentration of meloxicam, ketoprofen, or tolfenamic acid were obtained at 0 (pre-dose) hours, and 1, 2, 4, 6, and 24 hours after dosing. The doses of the transdermal formulations described in Example 1 were given at twice the injectable doses on a mg/kg basis.

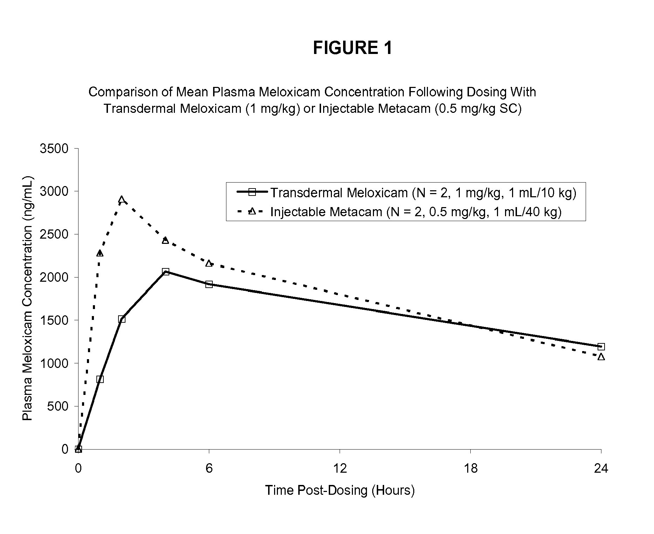

Figures 1 (meloxicam), 2 (ketoprofen), and 3 (tolfenamic acid) shows the plasma concentration of each active following transdermal and injectable dosing.

Surprisingly, for each active, the plasma concentration following transdermal dosing was similar to injectable dosing, thus illustrating the effectiveness of the currently described formulations at driving these NSAIDs through cattle hide and into the systemic circulation.

**Claims**

1. A liquid transdermal preparation for systemic administration of a non-steroidal anti-inflammatory compound selected from the following groups: oxicams, propionic acids, and anthranilic acids or a pharmacologically acceptable salt thereof to a non-human animal in a pharmaceutically acceptable carrier system comprising a solvent and at least one dermal penetration enhancer.

2. The transdermal liquid preparation of claim 1, wherein the non-steroidal anti-inflammatory compound is selected from meloxicam, ketoprofen, and tolfenamic acid or a pharmacologically acceptable salt thereof.

3. The transdermal liquid preparation of claim 1, wherein the penetration enhancer is selected from the group of terpenoids consisting of menthol, camphor, d-limonene, nerolidol, or 1-8 Cineole terpenoids, saturated or unsaturated fatty acid esters or diesters of propylene glycol, saturated or unsaturated fatty acid esters, diesters, or triesters of glycerol, saturated or unsaturated fatty alcohols, saturated or unsaturated fatty acids, xylene, isopropyl myristate, and mixtures thereof.

4. The transdermal liquid preparation of claim 1, wherein the solvent is selected from the group consisting of a pyrrolidone solvent, glycol ethers, N,N-dimethylacetamide, N,N-dimethylformamide, dimethyl sulfoxide (DMSO), acetone, glycerol formal, ethyl lactate, and/or mixtures thereof.

5. The transdermal liquid preparation of claim 4, wherein the glycol ether is selected from the group consisting of ethylene glycol monoethyl ether, diethylene glycol monoethyl ether, dipropylene glycol monoethyl ether and/or mixtures thereof.

6. The transdermal liquid preparation of claim 4, wherein the pyrrolidone solvent is selected from 2-pyrrolidone, N-methyl-2-pyrrolidone, and/or mixtures thereof.

7. The transdermal liquid preparation of claim 1, further comprising a second pharmaceutically active compound.

8. The transdermal liquid preparation of claim 7, wherein the second pharmaceutically active compound is selected from the group consisting of antimicrobials, anti-inflammatory agents, oxytocin, hormones for reproduction, growth enhancement compounds, physiologic intervention compounds, anxiolytic compounds, antihistamines, immune stimulants, and vaccines.

9. A transdermal liquid preparation of claim 1, comprising:

    a) at least 2% by wt of a dermal penetration enhancer;
    b) at least 0.5% of a non-steroidal anti-inflammatory compound selected from the following groups: oxicams, propionic acids, and anthranilic acids or a pharmacologically acceptable salt thereof; and
    c) up to 90% of a solvent.

10. The preparation of any of claims 1 to 9, wherein, upon transdermal administration of said preparation to a bovine animal, the non-steroidal anti-inflammatory compound has an absolute systemic bioavailability of at least 10%.

11. Use of the liquid transdermal preparation according to claims 1 to 10 for preparing a veterinary medical composition for transdermal treatment of systemic inflammatory diseases in mammals.

12. Use of the liquid transdermal preparation according to claims 1 to 10 in the manufacture of a medicament for inducing a systemic anti-inflammatory or antipyretic effect in a bovine animal after single dose transdermal administration to such animal.

13. Use of the liquid transdermal preparation according to claims 1 to 10, wherein the transdermal administration achieves an absolute systemic bioavailability of at least 10%.

# FIGURE 1

Comparison of Mean Plasma Meloxicam Concentration Following Dosing With
Transdermal Meloxicam (1 mg/kg) or Injectable Metacam (0.5 mg/kg SC)

# FIGURE 2

Comparison of Mean Plasma Ketoprofen Concentration Following Dosing With
Transdermal Ketoprofen (6 mg/kg) or Injectable Ketofen (3 mg/kg deep IM)

# FIGURE 3

Comparison of Mean Plasma Tolfenamic Acid Concentration Following Dosing With
Transdermal Tolfenamic Acid (4 mg/kg) or Injectable Tolfine (2 mg/kg IM)

Europäisches
Patentamt

European
Patent Office

Office européen
des brevets

# EUROPEAN SEARCH REPORT

Application Number

EP 15 15 1038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/27372 A (SQUIBB BRISTOL MYERS CO [US]; KANEKO TERUHISA [JP]; SHINOHARA YUKA [JP] 18 May 2000 (2000-05-18) | 1-3,7,9, 11 | INV. A61K9/00 A61K47/24 |
| Y | * claims 1-13 * <br> * examples 1-4 * | 1-13 | A61K47/10 A61K31/00 A61K9/08 |
| X | WO 00/53228 A (FARMIGEA SPA [IT]; SAETTONE MARCO FABRIZIO [IT]; GIANNACCINI BORIS [IT] 14 September 2000 (2000-09-14) | 1-4,9-11 | |
| Y | * examples 6,7 * <br> * page 44; table 9 * | 1-13 | |
| X | EP 0 331 382 A (PFIZER [US]) 6 September 1989 (1989-09-06) | 1-4,9-11 | |
| Y | * example 9 * <br> * page 2, line 1 - line 2 * <br> * page 2, line 34 - line 44 * <br> * page 3, line 17 - line 20 * | 1-13 | |
| X | YUAN Y ET AL: "Physicochemical Properties and Evaluation of Microemulsion Systems for Transdermal Delivery of Meloxicam", CHEMICAL RESEARCH IN CHINESE UNIVERSITIES, BEIJING, CN, vol. 23, no. 1, 1 January 2007 (2007-01-01), pages 81-86, XP022856002, ISSN: 1005-9040 [retrieved on 2007-01-01] | 1-3,9,10 | |
| Y | * page 81 * <br> * page 82, paragraph EXPERIMENTAL * | 1-13 | |
| X | US 2005/244485 A1 (HSU TSUNG-MIN [US] ET AL) 3 November 2005 (2005-11-03) | 1-3 | |
| Y | * example 1 * <br> * page 3, paragraph 32 - page 4, paragraph 36 * | 1-13 | |

TECHNICAL FIELDS
SEARCHED (IPC)

A61K

-/--

The present search report has been drawn up for all claims

| Place of search | Date of completion of the search | Examiner |
|---|---|---|
| Munich | 16 April 2015 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 15 15 1038

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CAFAGGI S ET AL: "An example of application of a mixture design with constraints to a pharmaceutical formulation", CHEMOMETRICS AND INTELLIGENT LABORATORY SYSTEMS, ELSEVIER SCIENCE PUBLISHERS B.V. AMSTERDAM, NL, vol. 65, no. 1, 28 January 2003 (2003-01-28), pages 139-147, XP004397731, ISSN: 0169-7439 * the whole document * | 1-13 | |
| Y | US 6 045 827 A (RUSSELL MERI CHARMYNE [US]) 4 April 2000 (2000-04-04) * column 11, line 50 - column 13, line 25 * * claims 1-19 * | 1-13 | |

TECHNICAL FIELDS SEARCHED (IPC)

The present search report has been drawn up for all claims

| Place of search | Date of completion of the search | Examiner |
|---|---|---|
| Munich | 16 April 2015 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 15 1038

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2015

| Patent document cited in search report | | | Publication date | Patent family member(s) | | | Publication date |
|---|---|---|---|---|---|---|---|
| WO 0027372 | A | | 18-05-2000 | AT | 401869 | T | 15-08-2008 |
| | | | | CA | 2348869 | A1 | 18-05-2000 |
| | | | | CN | 1354655 | A | 19-06-2002 |
| | | | | DK | 1126830 | T3 | 25-08-2008 |
| | | | | EP | 1126830 | A1 | 29-08-2001 |
| | | | | ES | 2308860 | T3 | 01-12-2008 |
| | | | | ID | 29530 | A | 06-09-2001 |
| | | | | JP | 2000143540 | A | 23-05-2000 |
| | | | | MY | 130857 | A | 31-07-2007 |
| | | | | PT | 1126830 | E | 06-08-2009 |
| | | | | WO | 0027372 | A1 | 18-05-2000 |
| WO 0053228 | A | | 14-09-2000 | AU | 3324000 | A | 28-09-2000 |
| | | | | EP | 1159009 | A2 | 05-12-2001 |
| | | | | IT | RM990151 | A1 | 11-09-2000 |
| | | | | WO | 0053228 | A2 | 14-09-2000 |
| EP 0331382 | A | | 06-09-1989 | CA | 1331137 | C | 02-08-1994 |
| | | | | DE | 68902753 | D1 | 15-10-1992 |
| | | | | DE | 68902753 | T2 | 21-01-1993 |
| | | | | DK | 93289 | A | 30-08-1989 |
| | | | | EP | 0331382 | A2 | 06-09-1989 |
| | | | | ES | 2051998 | T3 | 01-07-1994 |
| | | | | FI | 890930 | A | 30-08-1989 |
| | | | | GR | 3005657 | T3 | 07-06-1993 |
| | | | | IE | 61946 | B1 | 30-11-1994 |
| | | | | JP | H023613 | A | 09-01-1990 |
| | | | | JP | H0768147 | B2 | 26-07-1995 |
| | | | | PT | 89837 | A | 04-10-1989 |
| US 2005244485 | A1 | | 03-11-2005 | AU | 2004281766 | A1 | 28-04-2005 |
| | | | | CA | 2542753 | A1 | 28-04-2005 |
| | | | | CN | 1886105 | A | 27-12-2006 |
| | | | | EP | 1682060 | A1 | 26-07-2006 |
| | | | | JP | 2007510628 | A | 26-04-2007 |
| | | | | US | 2005244485 | A1 | 03-11-2005 |
| | | | | US | 2011178044 | A1 | 21-07-2011 |
| | | | | WO | 2005037157 | A1 | 28-04-2005 |
| US 6045827 | A | | 04-04-2000 | AT | 326948 | T | 15-06-2006 |
| | | | | AU | 5435998 | A | 10-06-1998 |
| | | | | BR | 9714573 | A | 23-07-2002 |
| | | | | CA | 2273183 | A1 | 28-05-1998 |
| | | | | DE | 69735949 | T2 | 04-01-2007 |
| | | | | EP | 0946147 | A1 | 06-10-1999 |
| | | | | ES | 2263182 | T3 | 01-12-2006 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 15 15 1038

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2015

| Patent document cited in search report | Publication date | Patent family member(s) | Publication date |
|---|---|---|---|
| | | JP 2001509136 A | 10-07-2001 |
| | | US 5891472 A | 06-04-1999 |
| | | US 6045827 A | 04-04-2000 |
| | | US 6287601 B1 | 11-09-2001 |
| | | WO 9822090 A1 | 28-05-1998 |

EPO FORM P0459

For more details about this annex : see Official Journal of the European Patent Office, No. 12/82

**EP 2 889 028 A1**

**Patent documents cited in the description**

- US 4235892 A